# EUROPÄISCHE PATENTANMELDUNG

(11) **EP 4 806 470 A2**
(43) Veröffentlichungstag der Anmeldung: **16.09.2026**
(21) Anmeldenummer: 26164261.5
(22) Anmeldetag: 12.03.2026
(51) Int. Cl.: A61L 2/208, A61L 2/10, A61L 2/087, A61L 2/101, B65B 55/02, B65B 55/10, B65B 55/12, B65G 17/16, A61L 103/00

(54) **VORRICHTUNG ZUM STERILISIEREN VON BEHÄLTNISSEN, VORRICHTUNG ZUM TRANSPORTIEREN VON BEHÄLTNISSEN, HALTEEINRICHTUNG FÜR BEHÄLTNISSE UND VERFAHREN**

(30) Priorität: 13.03.2025 DE 102025109606
(71) Anmelder: Krones AG, 93073 Neutraubling (DE)
(72) Erfinder: Geltinger, Florian, 93073 Neutraubling (DE); Spitzer, Thomas, 93073 Neutraubling (DE); Kindl, Norbert, 93073 Neutraubling (DE)
(74) Vertreter: Hannke Bittner & Partner mbB Regensburg

(57) **Zusammenfassung**

Vorrichtung (1) zum Behandeln von Behältnissen, insbesondere von Kunststoffbehältnissen (10) und insbesondere von Kunststoffvorformlingen (10) mit einer Transporteinrichtung (2) zum Transportieren der Kunststoffbehältnisse (10) entlang eines vorgegebenen Transportpfads und mit wenigstens einer Beaufschlagungseinrichtung (4), welche dazu geeignet und bestimmt ist, die von der Transporteinrichtung (2) transportierten Kunststoffbehältnisse (10) mit einem fließfähigen Sterilisationsmedium zu beaufschlagen, wobei die Transporteinrichtung (2) einen bewegbaren und bevorzugt um eine vorgegebene Drehachse drehbaren Transportträger (22) aufweist, an welchem eine Vielzahl von Halteeinrichtungen (6) zum Halten der Kunststoffbehältnisse (10) angeordnet ist, dadurch gekennzeichnet, dass die Halteeinrichtung (6) eine Greifeinrichtung (62) aufweist, welche dazu geeignet und bestimmt ist die Kunststoffbehältnisse wenigstens in einem ersten Abschnitt der Kunststoffbehältnisse zu kontaktieren sowie eine Rückhalteeinrichtung (64), welche dazu geeignet und bestimmt ist, die Kunststoffbehältnisse in einem zweiten Abschnitt der Kunststoffbehältnisse (10) zu kontaktieren, wobei der erste Abschnitt zum zweiten Abschnitt beabstandet ist, wobei die Greifeinrichtung relativ zur Transporteinrichtung feststehend ist und die Rückhalteeinrichtung relativ zur Transporteinrichtung beweglich ist.

## Beschreibung

Die vorliegende Erfindung bezieht sich auf eine Vorrichtung und ein Verfahren zum Behandeln und insbesondere zum Sterilisieren von Behältnissen, insbesondere von Kunststoffbehältnissen und insbesondere von Kunststoffvorformlingen. Daneben bezieht sich die vorliegende Erfindung auch auf eine Transporteinrichtung zum Transportieren von Kunststoffbehältnissen und insbesondere von Kunststoffvorformlingen sowie eine Halteeinrichtung zum Halten von Kunststoffbehältnissen und insbesondere von Kunststoffvorformlingen.

Aus dem Stand der Technik sind unterschiedliche Vorrichtungen und Verfahren zum Sterilisieren von Kunststoffvorformlingen bekannt. Üblicherweise wird bei der Herstellung von Kunststoffflaschen zunächst ein Kunststoffvorformling erwärmt und anschließend mit einer Umformungseinrichtung wie beispielsweise einer Streckblasmaschine zu der Kunststoffflasche, wie etwa einem Getränkebehältnis umgeformt.

Dabei ist es ebenfalls aus dem Stand der Technik bekannt, dass die Kunststoffvorformlinge sterilisiert werden und insbesondere mit einem Sterilisationsmittel wie (insbesondere gasförmigen) Wasserstoffperoxid zu ihrer Sterilisation beaufschlagt werden. Es ist jedoch auch eine Sterilisation mittels elektromagnetischer Strahlung denkbar auf welche die vorliegende Erfindung prinzipiell ebenfalls anwendbar ist.

Zu diesem Zweck sind aus dem Stand der Technik Sterilisationsmodule bekannt. So ist beispielsweise aus dem Stand der Technik der Anmelderin ein Sterilisationsmodul mit drei Behandlungsternen bekannt, welche jeweils Handlingsklammern zum Greifen der Behältnisseaufweisen. Es wird darauf hingewiesen, dass im Rahmen der vorliegenden Beschreibung insbesondere auch die Kunststoffvorformlinge als Behältnisse angesehen werden.

Durch steigende Ausbringleistungen und Behandlungszeiten bzw. Lograten werden deutlich mehr derartige Handlingsklammern notwendig. Dadurch ergeben sich steigende Kosten und auch die Inbetriebnahme und Wartung wird aufwendiger.

Der vorliegenden Erfindung liegt daher die Aufgabe zugrunde, eine Sterilisationsvorrichtung und ein Verfahren zur Verführung zu stellen, welche höhere Ausgangleistungen und höhere Behandlungszeiten ermöglichen. Daneben soll auch eine Transporteinrichtung zum Transportieren von Behältnissen und insbesondere Kunststoffvorformlingen zur Verfügung gestellt werden, welche einen höheren Durchsatz erlauben. Daneben soll auch eine Halteeinrichtung zum Halten von Behältnissen und insbesondere von Kunststoffvorformlingen zur Verfügung gestellt werden, welche ebenfalls einen höheren Durchsatz ermöglicht.

Aus dem Stand der Technik sind auch Transporteinrichtungen bekannt, welche Beispiel Sägezahnsterne aufweisen, wobei jede Ausnehmung eines solchen Sägezahnsterns einen Kunststoffverformung trägt. Damit die Kunststoffvorformlinge nicht aus dem Sägezahlstern bzw. den Ausnehmungen herausfallen ist ein außerhalb des Sägezahnsterns angeordneter Führungsbogen vorgesehen. Dieser behindert jedoch teilweise die Zugänglichkeit der Behältnisse bzw. Kunststoffvorformlinge, insbesondere wenn diese beispielsweise sterilisiert werden sollen.

Diese Transporteinrichtungen weisen weiterhin den Nachteil auf, dass sie insbesondere bei hoher Transportgeschwindigkeit unzuverlässig werden. Eine weitere der Erfindung zugrundeliegende Aufgabe besteht also darin, die Zuverlässigkeit derartiger Transporteinrichtungen zu verbessern.

Diese Aufgaben werden durch die Gegenstände der unabhängigen Patentansprüche gelöst. Vorteilhafte Ausführungsformen und Weiterbildungen sind Gegenstand der Unteransprüche.

Eine erfindungsgemäße Vorrichtung zum Behandeln von Behältnissen und insbesondere zum Sterilisieren von Behältnissen, insbesondere von Kunststoffbehältnissen und insbesondere von Kunststoffvorformlingen weist eine Transporteinrichtung zum Transportieren der Kunststoffbehältnisse entlang eines vorgegebenen Transportpfads auf. Weiterhin weist die Vorrichtung bevorzugt wenigstens eine Beaufschlagungseinrichtung auf, welche dazu geeignet und bestimmt ist, die von der Transporteinrichtung transportierten Kunststoffbehältnisse mit einem fließfähigen und insbesondere gasförmigen Sterilisationsmedium (oder ggfs mit elektromagnetischer Strahlung) und/oder einer Beschichtung und/oder einer elektromagnetischen Strahlung zu beaufschlagen, wobei die Transporteinrichtung einen bewegbaren und bevorzugt um eine vorgegebene Drehachse drehbaren Transportträger aufweist, an welchem eine Vielzahl von Halteeinrichtungen zum Halten der Kunststoffbehältnisse angeordnet ist. Anstelle der Beaufschlagungseinrichtung könnten auch andere Behandlungseinrichtungen vorgesehen sein, welche an den Kunststoffbehältnissen Behandlungen vornehmen.

Die Erfindung wird unter Bezugnahme auf einen Behandlungsvorgang in Form eines Sterilisationsvorgangs beschrieben. Hierfür ist die Erfindung auch in besonders vorteilhafter Weise geeignet. Es wären aber auch andere Behandlungsvorgänge denkbar, wie etwa Spülvorgänge, Bedruckungsvorgänge, Beschichtungsvorgänge oder Erwärmungsvorgänge oder Abkühlvorgänge. Insbesondere handelt es sich jedoch um Behandlungsvorgänge, bei denen wenigstens auf einen Abschnitt des Kunststoffbehältnisses und/oder Kunststoffvorformlings eingewirkt wird.

Erfindungsgemäß weist die Halteeinrichtung bzw. weisen die Halteeinrichtungen jeweils eine Greifeinrichtung auf, welche dazu geeignet und bestimmt ist die Kunststoffbehältnisse wenigstens in einem ersten Abschnitt der Kunststoffbehältnisse zu kontaktieren.

Weiterhin weist die Halteeinrichtung eine Rückhalteeinrichtung oder weisen die Halteeinrichtungen jeweils Rückhalteeinrichtungen auf, welche dazu geeignet und bestimmt ist, die Kunststoffbehältnisse in einem zweiten Abschnitt der Kunststoffbehältnissen zu kontaktieren, wobei der erste Abschnitt zum zweiten Abschnitt beabstandet ist, wobei die Greifeinrichtung relativ zur Transporteinrichtung feststehend ist und die Rückhalteeinrichtung relativ zur Transporteinrichtung beweglich ist
Bei der Greifeinrichtung kann es sich beispielsweise um einen (ersten) Greifarm einer Greifklammer handeln. Die Rückhalteeinrichtung kann beispielsweise auch wie ein zweiter Greifarm einer Greifklammer wirken.

Bevorzugt ist die Rückhalteeinrichtung derart gestaltet, dass sie das Kunststoffbehältnis rückhalten kann und/oder verhindern kann, dass das Kunststoffbehältnis sich von der Greifeinrichtung löst.

Bevorzugt ist der erste Abschnitt zu dem zweiten Abschnitt in einer Umfangsrichtung der Kunststoffbehältnisse beabstandet.

Bevorzugt umgibt die Greifeinrichtung das zu haltende Kunststoffbehältnis und/oder den zu haltenden ersten Abschnitt in einem ersten vorgegebenen Umfangswinkel und die Rückhalteeinrichtung umgibt das zu haltende Kunststoffbehältnis und insbesondere den zweiten Abschnitt in einem zweiten vorgegebenen Umfangswinkel.

Bevorzugt ist dabei der zweite Umfangswinkel größer als der erste Umfangswinkel.

Bevorzugt ist der erste Umfangswinkel größer als 20°, bevorzugt größer als 30°, bevorzugt größer als 40°, bevorzugt größer als 50°, bevorzugt größer als 60°; bevorzugt größer als 70°, und bevorzugt größer als 80° (insbesondere bezüglich der Längsrichtung des zu haltenden Kunststoffbehältnisses.

Bevorzugt ist der erste Umfangswinkel kleiner als 150°, bevorzugt kleiner als 140°, bevorzugt kleiner als 130°, bevorzugt kleiner als 120°, bevorzugt kleiner als 110°; bevorzugt kleiner als 100°. (insbesondere bezüglich der Längsrichtung des zu haltenden Kunststoffbehältnisses

Bevorzugt ist der zweite Umfangswinkel größer als 40°, bevorzugt größer als 50°, bevorzugt größer als 60°, bevorzugt größer als 70°, bevorzugt größer als 80°; bevorzugt größer als 90°, und bevorzugt größer als 100° und bevorzugt größer als 120° (insbesondere bezüglich der Längsrichtung des zu haltenden Kunststoffbehältnisses.

Bevorzugt ist der zweite Umfangswinkel kleiner als 170°, bevorzugt kleiner als 160°, bevorzugt kleiner als 150°, bevorzugt kleiner als 140°, bevorzugt kleiner als 130°. (insbesondere bezüglich der Längsrichtung des zu haltenden Kunststoffbehältnisses

Bevorzugt sind der erste Umfangswinkel und der zweite Umfangswinkel in Summe größer als 180°, bevorzugt größer als 190°, bevorzugt größer als 200° und besonders bevorzugt größer als 210° und besonders bevorzugt größer als 220° und besonders bevorzugt größer als 230°. Bevorzugt sind der erste Umfangswinkel und der zweite Umfangswinkel in Summe kleiner als 320°, bevorzugt kleiner als 310°, bevorzugt kleiner als 300° und besonders bevorzugt kleiner als 290°.

Es wäre jedoch auch zusätzlich oder alternativ denkbar, dass der zweite Abschnitt in einer Längsrichtung der Kunststoffbehältnisse von dem ersten Abschnitt beabstandet ist.

Bei einer weiteren Ausführungsform wäre es auch möglich, dass eine Greifeinrichtung mit wenigstens zwei Greifarmen (welche jedoch auch einstückig miteinander ausgebildet sein können) zusätzlich zu der Rückhalteeinrichtung vorgesehen ist, wobei wenigstens ein erster Abschnitt der Behältnisse und insbesondere Kunststoffvorformlinge zwischen diesen Greifarmen und insbesondere in einer zwischen diesen zwei Greifarmen gebildeten Ausnehmung aufnehmbar ist.

Es werden im Folgenden zwei Ausgestaltungen beschrieben. Bei beiden Ausführungsformen ist eine Greifeinrichtung mit wenigstens einem Greifelement (bzw. Greifarm) und einer Rückhalteeinrichtung vorgesehen, wobei das Greifelement relativ zur Transporteinrichtung (und/oder dem zu greifenden Kunststoffbehältnis und/oder Kunststoffvorformling) feststehend ist und die Rückhalteeinrichtung relativ zur Transporteinrichtung (und/oder dem zu greifenden Kunststoffbehältnis und/oder Kunststoffvorformling) beweglich und insbesondere bezüglich einer vorgegebenen Schwenkachse schwenkbar ist.

Bei einer ersten Ausführungsform ist ein Greifelement bzw. eine Greifeinrichtung feststehend und eine Rückhalteeinrichtung beweglich (bezüglich der Transporteinrichtung und/oder dem Kunststoffbehältnis).

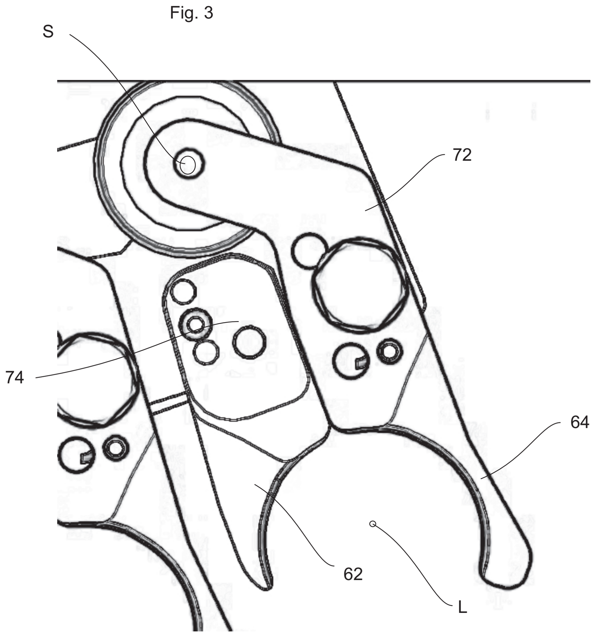

Die Rückhalteeinrichtung weist bevorzugt einen Hinterschnitt bzw. eine Ausnehmung auf, welcher in einer Rückhalteposition das Kunststoffbehältnis bzw. den Kunststoffvorformling in einem zweitem Abschnitt hält. Bevorzugt verläuft dabei eine Schwenkachse der Rückhalteleeinrichtung parallel zu einer Längsrichtung des Kunststoffvorformlings. Dies Ausführungsform ist in Fig. 3 untenstehend erläutert.

Bevorzugt bildet die Greifeinrichtung keinen Hinterschnitt aus.

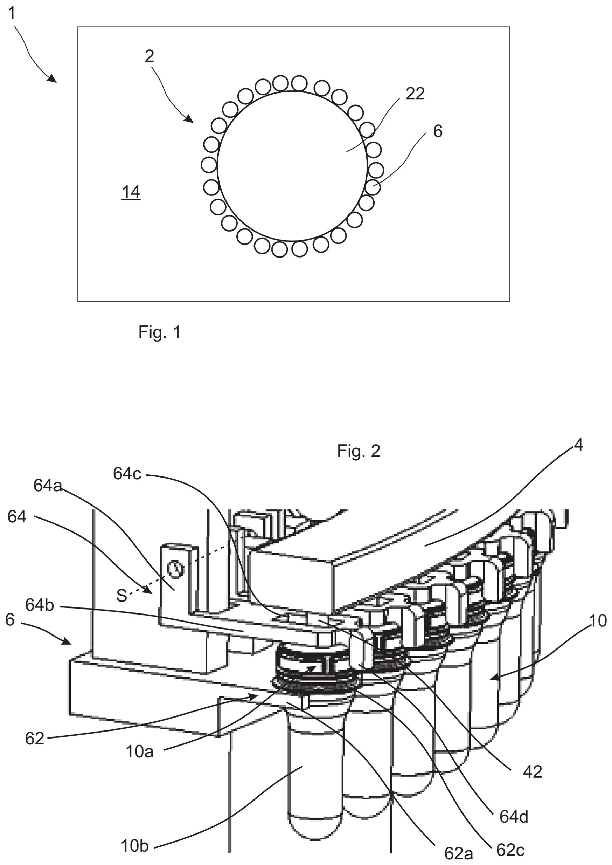

Bei einer weiteren Ausführungsform bildet eine Greifeinrichtung (insbesondere mittels zwei Greifarmen) eine Tasche zur Aufnahme der Kunststoffbehältnisse aus. Bevorzugt kontaktiert die Rückhalteeinrichtung das Kunststoffbehältnis an einem Abschnitt, der in Längsrichtung von dem ersten Abschnitt beabstandet ist. Besonders bevorzugt ist eine Schwenkachse der Rückhalteeinrichtung hier senkrecht zu der Längsrichtung der Kunststoffbehältnisse bzw. Kunststoffvorformlinge. Diese Ausführungsform ist in Fig. 2 untenstehend erläutert

Diese beiden Ausführungsformen werden untenstehend genauer erläutert.

Bei der Längsrichtung bzw. der Längsachse handelt es sich insbesondere um eine Achse, welche sich von einer Mündung des Behältnisses zu einem Boden des Behältnisses (und insbesondere im Falle eines Kunststoffvorformlings zu einer Bodenkuppe) erstreckt.

Auf diese Weise kann ein Herausfallen der Kunststoffbehältnisse aus der zwischen den Greifarmen gebildeten Ausnehmung verhindert werden. Damit ist bevorzugt die Rückhalteeinrichtung auch ein Bestandteil der Halteeinrichtungen. Bevorzugt weist jede Halteeinrichtungen eine Rückhalteeinrichtung auf. Damit entspricht bevorzugt die Anzahl der Rückhalteeinrichtungen auch der Anzahl der Halteeinrichtungen.

Bevorzugt handelt es sich bei dem fließfähigen Sterilisationsmedium um H₂O₂. Es kann jedoch auch eine andere Substanz wie Peressigsäure eingesetzt werden, und es wäre auch möglich, anstelle eines fließförmigen d.h. insbesondere flüssigen oder gasförmigen Sterilisationsmediums eine sterilisierende Strahlung zum Sterilisieren einzusetzen, beispielsweise UV-Strahlung, Laserstrahlung, oder Elektronenstrahlung. Die Anmelderin behält sich daher vor, auch für derartige Ausgestaltungen Schutz zu beanspruchen.

Bei einer weiteren vorteilhaften Ausführungsform weist die Vorrichtung einen Behandlungsraum auf, innerhalb dessen die Kunststoffbehältnisse während ihrer Sterilisation transportiert werden bzw. transportierbar sind.

Bevorzugt sind die Kunststoffbehältnissen entlang eines kreisförmigen oder kreissegmentförmigen Transportpfads transportierbar.

Bevorzugt ist auch die Beaufschlagungseinrichtung - vorteilhaft vollständig - innerhalb des Behandlungsraums angeordnet. Bei einer weiteren vorteilhaften Ausführungsform sind innerhalb des Behandlungsraums auch noch wenigstens ein bevorzugte wenigstens zwei weitere Transporteinrichtungen und insbesondere Transportsterne angeordnet.

Bevorzugt ist der Behandlungsraum als Reinraum ausgebildet, innerhalb dessen sterile Bedingungen aufrecht erhalten werden können.

Besonders bevorzugt bilden die Halteeinrichtungen und insbesondere die Greifeinrichtungen (der Halteeinrichtungen) jeweils Transporttaschen auf, welche zur Aufnahme jeweils eines Kunststoffbehältnisses bzw. eines (Bereichs eines) Mündungsabschnitts eines Kunststoff Behältnisses geeignet und bestimmt sind.

Bevorzugt sind die Greifeinrichtungen derart ausgebildet, dass sie die Behältnisse und insbesondere die Kunststoffvorformlinge in deren Längsrichtung abstützen. Insbesondere werden dabei die Behältnisse und insbesondere die Kunststoffvorformlingen an ihren Tragringen abgestützt.

Bei einer weiteren bevorzugten Ausführungsform ist die Beaufschlagungseinrichtung derart angeordnet, dass sie im Wesentlichen und/oder zumindest auch eine Beaufschlagung eines Innenraums der Kunststoffbehältnisse mit dem fließfähigen Sterilisationsmedium ermöglicht. So ist es möglich, dass das Sterilisationsmedium zunächst einen Innenraum der Kunststoffbehältnisse und insbesondere Kunststoffvorformlinge beaufschlagt und anschließend auch äußere Abschnitte des Kunststoffbehältnisses und insbesondere Kunststoffvorformlings wie insbesondere einen Gewinde- und/oder Mündungsbereich.

Insbesondere ist dabei die Beaufschlagungseinrichtung oberhalb der Kunststoffbehältnisse und/oder oberhalb des Transportpfads der Kunststoffbehältnisse angeordnet.

Bevorzugt weist die Beaufschlagungseinrichtung eine Vielzahl von Beauftragungsdüsen auf, welche dazu geeignet und bestimmt sind, einen Innenraum der Kunststoffbehältnisse mit dem Sterilisationsmedium zu beaufschlagen. Bevorzugt weisen dabei diese Beaufschlagungseinrichtungen jeweils zueinander den gleichen Abstand und/oder die gleiche Teilung auf wie die Halteeinrichtungen.

Bei einer bevorzugten Ausführungsform ist die Beaufschlagungseinrichtung und/oder sind diese Beaufschlagungsdüsen stationär angeordnet und die Kunstbehältnisse bewegen sich relativ zu diesen Beaufschlagungsdüsen.

Bei einer weiteren bevorzugten Ausführungsform bewegen sich die Beaufschlagungseinrichtungen und/oder Beaufschlagungsdüsen mit den Kunststoffbehältnissen mit. Bevorzugt sind die Beaufschlagungsdüsen fest mit der Transporteinrichtung, beispielsweise einem Transportstern verbunden und bewegen sich mit dem jeweiligen Kunststoffbehältnis und insbesondere dem jeweiligen Kunststoffvorformling mit.

Bei einer weiteren bevorzugten Ausführungsform weist die Vorrichtung eine Zuführeinrichtung auf, welche der Transporteinrichtung die Kunststoffbehältnissen zuführt. Besonders bevorzugt handelt es sich bei dieser Zuführeinrichtung um einen sog. Teilungsverzugsstern, der dazu geeignet und bestimmt ist, eine Teilung zwischen den von ihm transportierten Kunststoffbehältnissen zu ändern.

Besonders bevorzugt weist diese Zuführeinrichtung eine Vielzahl von Halteeinrichtungen auf, welche zum Halten der Behältnisse und insbesondere der Kunststoffvorformlinge geeignet und bestimmt sind. Bevorzugt weichen diese Halteeinrichtungen jeweils Halteelemente auf, die dazu geeignet und bestimmt sind, die einzelnen Behältnisse in einem Mündungsbereich und insbesondere in einem Bereich oberhalb des Tragrings der Kunststoffbehältnisse und insbesondere der Kunststoffvorformlinge zu halten und/oder zu fixieren. Auf diese Weise ist eine einfache Übergabe an die Halteeinrichtungen der Transporteinrichtung möglich.

Es wird daher vorgeschlagen, dass die Kunststoffbehältnisse und insbesondere die Kunststoffvorformlinge insbesondere durch die Zuführeinrichtung, bei der sich insbesondere um einen Teilungsverzugsstern handelt, an die Transporteinrichtung bzw. die einzelnen Halteeinrichtungen eingegeben werden und auch von dort wieder entnommen werden.

Besonders bevorzugt weist die Vorrichtung auch eine Abführeinrichtung auf, welche dazu geeignet und bestimmt ist, von der Transporteinrichtung transportierte und auch vorteilhaft bereits sterilisierte Kunststoffbehältnisse wieder von der Transporteinrichtung abzuführen.

Dabei kann es sich bevorzugt auch bei dieser Abführeinrichtung um einen Teilungsverzugsstern handeln. Es wäre jedoch auch denkbar, dass lediglich ein Teilungsverzugstern vorgesehen ist, der sowohl die Kunststoffbehältnisse der Transporteinrichtung zuführt als auch von der Transporteinrichtung die Kunststoffbehältnisse abführt.

Bevorzugt erfolgt in der Transporteinrichtung, die bevorzugt als Taschenstern ausgeführt ist, die Entkeimung insbesondere mittels gasförmigem H₂O₂.

Besonders bevorzugt greift die Transporteinrichtung insbesondere die einzelnen Halteeinrichtungen die Kunststoffbehältnisse jeweils unterhalb deren Tragring.

Besonders bevorzugt ist eine Teilung zwischen zwei aufeinanderfolgenden Halteeinrichtungen insbesondere zwischen den von den Halteeinrichtungen ausgebildeten Ausnehmungen bzw. Taschen kleiner als der 2,5 fache Tragringdurchmesser der Kunststoffvorformlinge, bevorzugt kleiner als der zweifache Tragringdurchmesser und bevorzugt bevorzugt kleiner als der 1,5 - fache Tragringdurchmesser.

Bei einer bevorzugten Ausführungsform weist die Ausnehmung bzw. die Aufnahmetasche eine Öffnung und insbesondere eine halbkreisförmige Öffnung auf. Besonders bevorzugt ist diese Öffnung tangential insbesondere über die Mitte des zu haltenden Kunststoffbehältnisses und/oder Kunststoffvorformlings und insbesondere über die Mitte des zu haltenden Abschnitts des Kunststoffbehältnisses verlängert.

Bevorzugt wird der Kunststoffvorformling bzw. das Kunststoffbehältnissen über ein verschieb- oder bewegbares Element, insbesondere die oben erwähnte Rückhalteeinrichtung vor einem Herausfallen gesichert.

Die vorliegende Erfindung ist weiterhin auf eine Transporteinrichtung zum Transportieren von Behältnissen, insbesondere von Kunststoffbehältnissen und insbesondere von Kunststoffvorformlingen gerichtet. Dabei weist die Transporteinrichtung einen um bewegbaren und insbesondere um eine vorgegebene Drehachse drehbaren Transportträger auf, an welchem eine Vielzahl von Halteeinrichtungen zum Halten der Behältnisse angeordnet ist.

Erfindungsgemäß weisen die Halteeinrichtungen jeweils (wenigstens) eine Greifeinrichtung auf, welche dazu geeignet und bestimmt ist die Kunststoffbehältnisse wenigstens in einem ersten Abschnitt der Kunststoffbehältnisse zu kontaktieren.

Weiterhin weist die Halteeinrichtung und weisen bevorzugt die Halteeinrichtungen jeweils eine Rückhalteeinrichtung auf, welche dazu geeignet und bestimmt ist, die Kunststoffbehältnisse in oder an einem zweiten Abschnitt der Kunststoffbehältnisse zu kontaktieren, wobei bevorzugt die Greifeinrichtung relativ zur Transporteinrichtung (und oder dem zu transportierenden Behältnis) feststehend ist und die Rückhalteeinrichtung relativ zur Transporteinrichtung beweglich ist um auf diese Weise die Behältnisse an der ersten Greifeinrichtung zu halten um auf diese Weise die Behältnisse an der Greifeinrichtung zu halten wobei die Rückhalteeinrichtung (64) ein zwischen einer Rückhalteposition und einer Freigabeposition bewegbares Rückhalteelement aufweist, wobei in der Rückhalteposition das Rückhalteelement das Behältnis und insbesondere einen Mündungsbereich des Behältnisses (10) an der Greifeinrichtung und dem Rückhalteelement hält und in der Freigabeposition das Behältnis aus einer an der ersten Greifeinrichtung gebildeten Ausnehmung und insbesondere ohne Kontakt mit dem Rückhalteelement heraustreten kann.

Es ist dabei möglich, dass eine Ausnehmung durch die Greifeinrichtung und eine weitere Greifeinrichtung oder auch durch die Greifeinrichtung und die Rückhalteeinrichtung gebildet wird. Auch kann die Greifeinrichtung selbst eine Ausnehmung aufweisen, an welcher der besagte Abschnitt des Kunststoffbehältnisses anliegen kann.

Es wird in diesem Zusammenhang darauf hingewiesen, dass der Greifarm oder die Greifarme die Behältnisse nicht notwendigerweise klammernd greifen müssen. Daher können die Greifarme auch als Haltearme bezeichnet werden. Auch ist es denkbar, dass die Behältnisse und insbesondere die Kunststoffvorformlinge ein (geringes) laterales Spiel in den Greifeinrichtungen haben.

Dabei weist die Rückhalteeinrichtung ein zwischen einer Rückhalteposition und einer Freigabeposition bewegbares Rückhalteelement auf, wobei in der Rückhalteposition des Rückhaltselements das Behältnis und insbesondere einen Mündungsbereich des Behältnisses zwischen den Greifarmen bzw. Haltearmen hält und/oder zurückhält und in der Freigabeposition das Behältnis und insbesondere der Kunststoffvorformling aus der zwischen den Greifarmen gebildeten Ausnehmung - insbesondere ohne Kontakt mit dem Rückhalteelement - heraustreten kann.

Es wird insbesondere ein gegenüber dem oder den Greifarmen bewegliches Rückhalteelement vorgeschlagen, welches zwischen wenigstens zwei Positionen verstellbar und oder schaltbar ist. Bevorzugt ist das Rückhalteelement bezüglich einer Schwenkachse schwenkbar, welche in der Längsrichtung oder parallel zu der Längsrichtung der zu haltenden Kunststoffbehältnisse verläuft.

Bei einer weiteren bevorzugten Ausführungsform ist das Rückhalteelement in der Art eines Greifarms ausgebildet und/oder weist das Rückhalteelement eine Ausnehmung auf, welche ebenfalls an einen Mündungsbereich des Kunststoffvorformlings anlegbar ist.

Bevorzugt weist das Rückhalteelement einen ersten Abschnitt und einen gegenüber dem ersten Abschnitt abgewinkelten zweiten Abschnitt auf. Bevorzugt ist der zweite Abschnitt als Ausleger ausgebildet. Insbesondere erstreckt sich wenigstens in der Rückhalteposition der Ausleger bzw. der zweite Abschnitt im Wesentlichen in der gleichen Richtung wie die Greifarme.

Bevorzugt stehen der erste und der zweite Abschnitt in einem Winkel zueinander, der zwischen 60° und 120°, bevorzugt zwischen 70° und 110°, besonders bevorzugt 80° und 100° und besonders bevorzugt bei 90° liegt.

Bei einer weiteren Position ist das Rückhalteelement zwischen der Rückhalteposition und der Freigabeposition um eine vorgegebene Schwenkachse schwenkbar. Bevorzugt ist das Rückhalteelement um einen Winkel schwenkbar, der größer ist als 10°, bevorzugt größer als 15°. Bevorzugt ist das Rückhalteelement um einen Winkel schwenkbar, der kleiner ist als 90°, bevorzugt kleiner als 80°, bevorzugt kleiner als 70°, bevorzugt kleiner als 60°, bevorzugt kleiner als 50° , bevorzugt kleiner als 40°.

Besonders bevorzugt verläuft diese Schwenkachse in eine Richtung, welche senkrecht (oder parallel) zu einer Längsrichtung eines von der Halteeinrichtungen zu haltenden oder gehaltenen Behältnisses verläuft. Bevorzugt verläuft diese Schwenkachse windschief zu einer Längsrichtung eines von der Halteeinrichtung zu haltenden oder gehaltenen Behältnisses.

Besonders bevorzugt verläuft die Schwenkachse in einer horizontalen Richtung, falls das Behältnis in einer aufrechten bzw. vertikalen Ausrichtung gehalten wird.

Bei einer weiteren bevorzugten Ausführungsform verläuft die Schwenkachse bei einer vertikalen Ausrichtung des Behältnisses und insbesondere Kunststoffbehältnisses oberhalb der Greifeinrichtung bzw. Halteeinrichtung und bevorzugt oberhalb eines von der Greifeinrichtung gehaltenen Kunststoffbehältnisses.

Es wäre jedoch auch möglich, dass das Rückhalteelement in einer vorgegebenen Richtung insbesondere in der Längsrichtung der Kunststoffbehältnisse verschiebbar ist, um es so von der Rückhaltsposition in die Freigabeposition (und umgekehrt) zu bewegen.

Bei einer weiteren bevorzugten Ausführungsform weist die Transporteinrichtung wenigstens eine Antriebseinrichtung zum Bewegen und insbesondere schwenken des oder der Rückhalteelemente auf.

Bevorzugt weist diese Antriebseinrichtung eine insbesondere stationäre Führungskurve auf, der gegenüber Führungsrollen abrollen, wobei bevorzugt jeder Halteeinrichtung und/oder jedem Rückhalteelement wenigstens eine Führungsrolle zugeordnet ist. Auf diese Weise kann durch den Führungskurvenantrieb das Schwenken und/oder Bewegen des Rückhalteelements zwischen der Rückhalteposition und der Freigabeposition bewirkt werden.

Es wären jedoch auch andere Antriebe denkbar, beispielsweise elektromotorische Antriebe.

Bei einer weiteren bevorzugten Ausführungsform weist die Transporteinrichtung ein Schaltmittel auf, welches dazu geeignet ist, die Rückhalteeinrichtung und/oder das Rückhalteelement zwischen der Rückhalteposition und der Freigabeposition zu schalten. Besonders bevorzugt ist wenigstens ein magnetisches Fixiermittel vorgesehen, welches dazu geeignet und bestimmt ist, die Rückhalteeinrichtung und/oder das Rückhalteelement in einer der beiden Positionen (wenigstens vorrübergehend) zu fixieren.

Mit anderen Worten weist die Transporteinrichtung bevorzugt wenigstens ein magnetisches Fixiermittel auf, welches dazu geeignet und bestimmt ist, die Rückhalteeinrichtung und/oder das Rückhalteelement in (wenigstens) einer der beiden Positionen zu fixieren und insbesondere vorübergehend zu fixieren. Bevorzugt wird durch eine Bewegung des Rückhaltemittels auch die Greifklammer zwischen einem geschlossenen Zustand (in welchem ein Behältnis gehalten wird) und einem geöffneten Zustand (in dem kein Behältnis gehalten wird) geschalten.

Im Stand der Technik werden üblicherweise Federmittel eingesetzt, um die Greifklammern in einen geöffneten oder einen geschlossenen Zustand zu halten. Im Rahmen der Erfindung wird vorgeschlagen, stattdessen ein oder mehrere magnetische Mittel vorzusehen, welche die Greifklammer in wenigstens einem der beiden Zustände halten (bzw. die Rückhalteeinrichtung und/oder das Rückhalteelement in einer der beiden Positionen zu halten).

Besonders bevorzugt weist das magnetische Fixiermittel wenigstens einen Permanentmagneten auf wobei bevorzugt der wenigstens eine Permanentmagnet an dem Rückhalteelement oder dem Rückhaltemittel und insbesondere an einem schwenkbaren Rückhalteelement angeordnet ist und bevorzugt mit diesem Rückhalteelement schwenkbar ist.

Bevorzugt weist das Schaltmittel wenigstens ein mit der Halteeinrichtung (mechanisch) gekoppeltes erstes magnetisches Element auf, wobei doch eine Bewegung dieses ersten magnetischen Elements ein Schalten des Rückhalteelements zwischen der ersten Position und der zweiten Position bewirkbar ist und das Schaltmittel weist bevorzugt wenigstens ein erstes bezüglich dem oder den Rückhalteelement(en) und bevorzugt räumlich stationär angeordnetes erstes magnetisches Stellelement auf, an welchem das Rückhalteelement und/oder die Rückhalteeinrichtung vorbeibewegbar ist, wobei eine während dieser Vorbeibewegung auftretende, insbesondere berührungslose magnetische Kraft und insbesondere eine magnetische Abstoßung(skraft) zwischen dem ersten magnetischen Element und dem ersten magnetischen Stellelement das Schalten des Rückhalteelements zwischen der ersten Position und der zweiten Position, d.h insbesondere zwischen der Rückhalteposition und der Freigabeposition bewirkt.

In dieser Ausgestaltung wird vorgeschlagen, dass das Schalten durch eine magnetische Kraft und insbesondere eine magnetische Abstoßung bewirkt wird. Auf diese Weise ist es möglich, eine entsprechendes Schalten des Elements auch bei sehr hohen Betriebsgeschwindigkeiten zu erreichen. Daneben ist diese Art des Antriebs frei von mechanischen Abnutzungserscheinungen, da die magnetische Kraftbeaufschlagung berührungslos erfolgt. Daneben kann diese Art des Schaltens geräuschärmer durchgeführt werden.

Besonders bevorzugt ist wenigstens einer der besagten Zustände ein Endzustand einer mechanischen Bewegung der Rückhalteeinrichtung bzw. des Rückhalteelements. Bei einer weiteren bevorzugten Ausführungsform sind beide Positionen jeweils Endpositionen einer mechanischen Bewegung der Rückhalteeinrichtung bzw. des Rückhalteelements. Auf diese Weise wird erreicht, dass die magnetische Abstoßung die Rückhalteeinrichtung bzw. das Rückhalteelement in einen mechanischen Endzustand (bezogen auf eine Bewegungsbahn des Rückhalteelements) drängt.

Diese Ausgestaltungen sind besonders vorteilhaft, wenn es sich bei der Rückhalteeinrichtung bzw. dem Rückhalteelement um einen Greifarm einer Greifklammer handelt, wie unten genauer beschrieben.

Bei einer weiteren bevorzugten Ausführungsform weist die Transporteinrichtung ein weiteres Fixiermittel auf, welches dazu geeignet und bestimmt ist, die Rückhalteeinrichtung und/oder das Rückhalteelement in der anderen der beiden Positionen zu fixieren.

Bei einer weiteren vorteilhaften Ausführungsform weist das Schaltmittel einen mit dem Rückhalteelement oder der Rückhalteeinrichtung mechanisch gekoppelten Schaltnocken auf, der bevorzugt von einem insbesondere stationär angeordneten Schaltmittel schaltbar ist. Bevorzugt ist dieser Schaltnocken drehbar oder schwenkbar. Dabei kann auch dieses weitere Schaltmittel ein magnetisches Element und insbesondere ein Permanentmagnet sein.

Weiterhin ist es möglich, dass ein Element des Schaltnockens in eine Ausnehmung eingreift, welche an dem Rückhalteelement ausgebildet ist. Auf diese Weise wird durch eine Drehung des Schaltnockens das Rückhalteelement bewegt.

So wäre es denkbar, dass die Antriebe berührungslos erfolgen, beispielsweise magnetisch
Daneben wäre es auch denkbar, dass eine Ansteuerung über einen vorgelagerten und/oder nachgelagerten (Transport)Stern; Dieser Stern könnte beispielsweise die Rückhaltelemente entsprechend kontaktieren und somit ansteuern.

Bei einer weiteren bevorzugten Ausführungsform ist die oder sind die Halteeinrichtungen jeweils separat und/oder getrennt an dem Transportträger angeordnet. Insbesondere sind die Halteeinrichtungen unabhängig voneinander von dem Träger entfernbar oder unabhängig voneinander an dem Transportträger anordenbar.

Bei einer weiteren bevorzugten Ausführungsform sind die Greifarme einteilig miteinander ausgebildet. Insbesondere handelt es sich bei den Greifeinrichtungen um passive Greifeinrichtungen, d. h. diese sind selbst bevorzugt nicht betätigbar bzw. permanent in der gleichen Greifstellung. Auf diese Weise kann die Vorrichtung einfacher gestaltet werden.

Bei einer weiteren vorteilhaften Ausführungsform weisen der oder die Greifarme jeweils einen gekrümmten Abschnitt auf und/oder bilden einen gekrümmten Abschnitt (oben als Ausnehmung bezeichnet) aus und insbesondere einen kreissegmentförmig gekrümmten Abschnitt. An diesem kreisförmig gekrümmten Abschnitt kann bevorzugt ein Bereich des gehaltenen Behältnisses und insbesondere ein Bereich des gehaltenen Kunststoffvorformlings anliegen.

Daneben weisen die Greifarme bevorzugt jeweils einen geradlinigen Abschnitt auf, der sich an den gekrümmten Abschnitt anschließt. Besonders bevorzugt bilden die Greifarme insgesamt einen halbkreisförmigen oder nahezu halbkreisförmigen Abschnitt aus.

Besonders bevorzugt bilden die beiden Greifarme gemeinsam eine U-förmige Ausnehmung aus, wobei die Ausnehmung an einer der Drehachse des Transporträgers zugewandten Seite insbesondere halbkreisförmig verläuft.

Bevorzugt verlaufen die geradlinigen Abschnitte zueinander parallel.

Besonders bevorzugt sind die Behältnisse und insbesondere Kunststoffvorformlinge ohne einen Widerstand in die Ausnehmung einführbar.

Durch das Vorsehen des Rückhaltelements kann erreicht werden, dass auf den beispielsweise bei Sägezahnsternen üblicherweise vorhandenen Führungsbogen verzichtet werden kann. Die Anmelderin hat herausgefunden, dass insbesondere beim Transport von Kunststoffvorformlingen die Gewichtsverhältnisse bzw. der Schwerpunkt so liegen, dass auch die Greifeinrichtung im Zusammenwirken mit dem Rückhaltelement ausreichend ist, um die Kunststoffvorformlinge auch bei höheren Drehgeschwindigkeiten des drehbaren Transportträgers sicher zu halten.

Besonders bevorzugt ist dabei das Rückhalteelement derart ausgebildet, dass auch der Ausleger die Behältnisse und insbesondere die Kunststoffvorformlinge kontaktiert (insbesondere mit seiner Unterseite) oder nahezu kontaktiert. Auf diese Weise kann auch ein Kippen der Behältnisse bzw. Kunststoffvorformlinge beispielsweise durch die Drehkraft verhindert werden.

Es ist dabei möglich und bevorzugt, dass die Behältnisse und insbesondere die Kunststoffvorformlinge durch ein Zusammenwirken der Greifeinrichtungen und der Rückhalteeinrichtungen mit einem leichten Spiel gehalten werden.

Bei einer weiteren vorteilhaften Ausführungsform weisen die Rückhalteelemente jeweils eine Öffnung auf, welche in der Rückhalteposition oberhalb der Mündung der Kunststoffbehältnisse bzw. der Kunststoffvorformlinge positioniert ist. Durch diese Öffnung hindurch kann beispielsweise eine Sterilisation der Kunststoffvorformlinge erfolgen. Auch können Beaufschlagungsdüsen durch diese Öffnungen geführt werden.

Damit weist bevorzugt die Ausnehmung bzw. die und insbesondere jede Aufnahmetasche der einzelnen Halteeinrichtungen eine halbkreisförmige Öffnung auf, die tangential insbesondere über der Mitte der Kunststoffvorformlinge hinaus verlängert ist.

Wie erwähnt, werden die Behältnisse und insbesondere die Kunststoffvorformlinge durch ein Halteelement, oben als Rückhalteelement bezeichnet, vor dem Herausfallen gesichert. Dieses Rückhalteelement kann dabei, wie oben erwähnt, vertikal verfahren oder bevorzugt von oben eingeschwenkt werden. Dabei liegt, wie oben erwähnt die Schwenkachse oder Drehachse dieses Rückhalteelements oberhalb einer Oberkante der Behältnisse und insbesondere der Kunststoffvorformlinge.

Wie oben erwähnt, kann ein Sterilisationsmedium durch eine feststehende Düse, insbesondere von oben zugeführt werden.

Die vorliegende Erfindung betrifft weiterhin eine Halteeinrichtung zum Halten von Behältnissen und insbesondere von Kunststoffbehältnissen und insbesondere von Kunststoffvorformlingen, wobei die Halteeinrichtung (wenigstens) eine Greifeinrichtung aufweist, welche dazu geeignet und bestimmt ist die Kunststoffbehältnisse wenigstens in einem ersten Abschnitt der Kunststoffbehältnisse zu kontaktieren um auf diese Weise die Behältnisse an der ersten Greifeinrichtung zu halten und weiterhin eine Rückhalteeinrichtung vorgesehen ist, welche dazu geeignet und bestimmt ist, die Behältnisse in einem zweiten Abschnitt der Behältnisse zu kontaktieren, wobei die Greifeinrichtung relativ zu der Halteeinrichtung und/oder relativ zu dem zu haltenden Behältnis feststehend ist und die Rückhalteeinrichtung relativ zu der Halteeinrichtung und/oder relativ zu dem zu haltenden Behältnis beweglich und insbesondere schwenkbar ist.

Weiterhin weist die Rückhalteeinrichtung ein zwischen einer Rückhalteposition und einer Freigabeposition bewegbares Rückhalteelement auf, wobei in der Rückhalteposition das Rückhalteelement das Behältnis und insbesondere einen Mündungsbereich des Behältnisses an der Greifeinrichtung und bevorzugt dem Rückhalteelement hält und in der Freigabeposition das Behältnis aus einer an der ersten Greifeinrichtung gebildeten Ausnehmung und insbesondere ohne Kontakt mit dem Rückhalteelement heraustreten kann.

Bevorzugt ist die Greifeinrichtung relativ zu dem Behältnis feststehend und die Rückhalteeinrichtung ist relativ zu dem (zu haltenden) Behältnis beweglich.

Bei einer weiteren vorteilhaften Ausführungsform weist die Rückhalteeinrichtung ein zwischen einer Rückhalteposition und einer Freigabeposition bewegbares Rückhalteelement auf, wobei in der Rückhalteposition des Rückhalteelements das Behältnis insbesondere in einem Mündungsbereich des Behältnisses zwischen der Greifeinrichtung und einem weiteren Greifarm hält und in der Freigabeposition das Behältnis, insbesondere der Kunststoffvorformling aus einer zwischen der Greifeinrichtung und dem weiteren Greifarm gebildeten Ausnehmung insbesondere ohne Kontakt mit dem Rückhaltelement heraustreten kann.

Bevorzugt handelt es sich um eine vertikale Ausrichtung der Behältnisse und insbesondere Kunststoffvorformlinge, bei welcher eine Mündung der Behältnisse und insbesondere Kunststoffvorformlinge oberhalb eines Grundkörpers der Behältnisse und insbesondere Kunststoffvorformlinge angeordnet ist.

Besonders bevorzugt weist die Halteeinrichtung ein Befestigungselement auf, welches ein Befestigen der Halteeinrichtung an einem Träger und insbesondere einem drehbaren Träger ermöglicht. Bevorzugt erlaubt dabei dieses Befestigungselement eine manuelle und/oder werkzeugfreie Montage und/oder Demontage der Halteeinrichtung an/von dem Transportträger.

Weiteren einer bevorzugten Ausführungsform bildet das Rückhalteelement auch einen Anlage- und/oder Abdeckabschnitt aus, der in der Position des Rückhalteelements entweder den Mündungsrand des Behältnisses und insbesondere des Kunststoffvorformlings kontaktiert oder unmittelbar benachbart zu diesem angeordnet ist.

Dieser Anlage- bzw. Abdeckabschnitt bewirkt, dass der von der Halteeinrichtung gehaltene Kunststoffvorformling nicht beispielsweise unter Wirkung einer Fliehkraft verschwenkt werden kann.

Besonders bevorzugt dient das Rückhalteelement in seiner Rückhalteposition daher auch als Niederhalteelement zum Niederhalten des Behältnisses und insbesondere des Kunststoffvorformlings. Bevorzugt verhindert das Niederhalteelement zumindest in der Rückhalteposition auch ein Austreten des Behältnisses und insbesondere des Kunststoffvorformlings in seiner Längsrichtung nach oben aus der Greifeinrichtung.

Besonders bevorzugt ist ein Abstand zwischen dem Abdeckabschnitt und dem Mündungsrand des Behältnisses geringer als 1 cm bevorzugt geringer als 0,5 cm bevorzugt geringer als 0,4 cm, bevorzugt geringer als 0,3cm, bevorzugt geringer als 0,2cm besonders bevorzugt geringer als 0,1cm.

Daneben wäre es auch möglich, dass das Rückhalteelement in der Rückhalteposition von oben den Tragering hält bzw. oberhalb des Tragrings angeordnet ist, damit auf diese Weise der Kunststoffvorformling nicht aus der Halteeinrichtungen entweichen kann.

So könnte auch das Rückhalteelement klammer- oder greifarmartig ausgeführt sein. In diesem Falle könnte bewirkt werden, dass im Wesentlichen die vollständige Mündung der Behältnisse und der Kunststoffvorformlinge für eine Sterilisation zugänglich bleibt.

Bei einer weiteren bevorzugten Ausführungsform ist die Halteeinrichtung, insbesondere in der oben beschriebenen Art zum Halten von Kunststoffvorformlingen und/oder zum Transportieren von Kunststoffvorformlingen geeignet und bestimmt.

Die vorliegende Erfindung ist weiterhin auf ein Verfahren zum Behandeln und insbesondere Sterilisieren von Behältnissen und insbesondere von Kunststoffvorformlingen gerichtet. Dabei transportiert eine Transporteinrichtung, welche bevorzugt einen um einen vorgegebenen Drehachse drehbaren Transporträger aufweist, an welchem eine Vielzahl von Halteeinrichtungen zum Halten der Kunststoffbehältnisse angeordnet ist oder wird, die Behältnisse entlang eines vorgegebenen Transportpfads.

Bevorzugt weisen die Halteeinrichtungen jeweils eine Greifeinrichtung auf, welche die Kunststoffbehältnisse wenigstens in einem ersten Abschnitt der Kunststoffbehältnisse kontaktieren sowie eine Rückhalteeinrichtung welche die Behältnisse an einem zweiten Abschnitt der Behältnissekontaktieren wobei der erste Abschnitt zum zweiten Abschnitt beabstandet ist und wobei die Greifeinrichtung relativ zur Transporteinrichtung feststehend ist und die Rückhalteeinrichtung relativ zur Transporteinrichtung beweglich ist wobei die Rückhalteeinrichtung ein zwischen einer Rückhalteposition und einer Freigabeposition bewegbares Rückhalteelement aufweist, wobei in der Rückhalteposition das Rückhalteelement das Behältnis und insbesondere einen Mündungsbereich des Behältnisses an der Greifeinrichtung hält und in der Freigabeposition das Behältnis aus einer an der ersten Greifeinrichtung gebildeten Ausnehmung und insbesondere ohne Kontakt mit dem Rückhalteelement heraustreten kann

Bei einer weiteren bevorzugten Ausführungsform weisen die Halteeinrichtungen jeweils eine erste Greifeinrichtung mit zwei Greifarmen auf (und/oder neben der ersten Greifeinrichtung einen zweiten Greifarm auf), wobei wenigstens ein erster Abschnitt der Behältnisse und insbesondere der Kunststoffbehältnisse zwischen der Greifeinrichtung und dem zweiten Greifarm aufgenommen wird und wobei jeweils die Rückhalteeinrichtung die Behältnisse in einem zweiten Abschnitt der Behältnisse, der in einer Längsrichtung der Behältnisse von dem ersten Abschnitt beabstandet ist, kontaktiert, um auf diese Weise die Behältnisse zwischen der Greifeinrichtung und dem Greifarm zu halten.

Dabei weist die Rückhalteeinrichtung ein zwischen einer Rückhalteposition und einer Freigabeposition bewegbares Rückhalteelement auf, wobei in der Rückhalteposition des Rückhalteelements das Behältnis, insbesondere das Kunststoffbehältnis und insbesondere den Kunststoffvorformling und insbesondere einen Mündungsbereich des Kunststoffvorformlings zwischen den Greifarmen (bzw. zwischen der Greifeinrichtung und dem Greifarm hält und wobei in einer Freigabeposition das Behältnis und insbesondere das Kunststoffbehältnis und insbesondere der Kunststoffvorformling aus einer zwischen den Greifarmen (bzw. zwischen der Greifeinrichtung und dem Greifarm) gebildeten Ausnehmung insbesondere ohne Kontakt mit dem Rückhalteelement heraustreten kann.

Insbesondere hält das Rückhalteelement das Behältnis und insbesondere das Kunststoffbehältnissen an dessen radialen Außenumfang. Es wäre jedoch auch möglich, dass ein Halt über eine Oberkante bzw. den Mündungsbereich des Behältnisses erfolgt. Es wäre jedoch auch möglich, dass das Rückhalteelement das Behältnis auf der gleichen Höhe hält wie die Greifeinrichtung. In diesem Fall wirkt das Rückhalteelement bevorzugt auch als zweiter Greifarm, der mit der Greifeinrichtung zusammenwirkt.

Bei einem weiteren vorteilhaften Verfahren werden die Behältnisse und insbesondere die Kunststoffvorformlinge während ihres Transports mit einem fließfähigen Sterilisationsmedium und/oder mit einer stabilisierenden Strahlung beaufschlagt. Besonders bevorzugt wird dabei auch ein Innenraum der Kunststoffvorformlinge sterilisiert.

Durch die vorliegende Erfindung wird insbesondere ein einfacheres und robusteres Handling insbesondere für Kunststoffvorformlinge in einem Behandlungsmodul gewährleistet.

Weitere Vorteile und Ausführungsformen ergeben sich aus den beigefügten Zeichnungen. Darin zeigen:
- Fig. 1: eine grob schematische Darstellung einer Vorrichtung zum Sterilisieren von insbesondere Kunststoffvorformlingen;
- Fig. 2: eine detailliertere Ansicht der erfindungsgemäßen Vorrichtung; und
- Fig. 3: eine Draufsicht auf eine Vorrichtung bzw. einen Abschnitt in einer weiteren erfindungsgemäßen Ausführungsform.

Fig. 1 zeigt eine grob schematische Darstellung einer Vorrichtung 1 zum Sterilisieren von Kunststoffvorformlingen in einer ersten Ausführungsform. Dabei ist innerhalb eines Behandlungsraums 14 eine Transporteinrichtung 2 zum Transportieren der (nicht gezeigten) Kunststoffvorformlinge vorgesehen. Diese Transporteinrichtung 2 weist einen drehbaren Transportträger 22 auf, an welchem bevorzugt äquidistant eine Vielzahl von Halteeinrichtungen 6 der oben beschriebenen Art angeordnet ist.

Fig. 2 zeigt eine Teilansicht der Transporteinrichtung 2 in einer ersten Ausführungsform. Man erkennt, dass hier eine Vielzahl von Kunststoffvorformlingen 10 gehalten werden kann, wobei die Kunststoffvorformlinge 10 jeweils einen Mündungsabschnitt 10b und einen Grundkörper 10b aufweisen.

Das Bezugszeichen 6 kennzeichnet eine Halteeinrichtung, welche hier zum Halten eines einzelnen Kunststoffvorformlings 10 dient. Diese Halteeinrichtung 6 weist eine Greifeinrichtung 62 auf, welche hier einen Greifarm 62a und einen zweiten (nicht gezeigten) Greifarm aufweist. In einer weiteren oben erläuterten Ausführungsform handelt es sich bei der Greifeinrichtung um einen (insbesondere bezüglich der Kunststoffvorformlinge 10 feststehenden Greifarm. Ein Kunststoffvorformling ist dabei unterhalb seines Tragerings 10c von dieser Greifeinrichtung haltbar oder abstützbar.

Das Bezugszeichen 64 kennzeichnet ein Rückhalteelement, welches ein ersten Abschnitt 64a und ein zweiten Abschnitt bzw. Ausleger 64b aufweist. Bei einer weiteren unten veranschaulichten Ausführungsform ist das Rückhalteelement als Greifarm ausgebildet.

Das Rückhaltselement 64 ist bezüglich einer Schwenk Achse S schwenkbar, und zwar von einer in Fig. 2 zwei gezeigten Rückhalteposition in eine Freigabeposition. Das Bezugszeichen 64d kennzeichnet einen Vorsprung der Rückhaltevorrichtung bzw. eine Rückhaltevorsprung, welcher dazu geeignet ist, Kunststoffvorformlinge 10 zurück zu halten, sodass dieser nicht aus der Greifeinrichtung heraustreten kann.

Das Bezugszeichen 64c kennzeichnet eine Öffnung, welche in dem Ausleger 64b angeordnet ist. Durch diese Öffnung kann beispielsweise eine Beaufschlagungsdüse 42 einer insgesamt mit 4 gekennzeichneten Beaufschlagungseinrichtung geführt werden.

Fig. 3 zeigt eine weitere Ausführungsform einer Halteeinrichtung. Dabei ist eine Greifeinrichtung 62 vorgesehen, welche hier jedoch in der Art eines Greifarms ausgebildet ist. Das Bezugszeichen 64 kennzeichnet die Rückhalteeinrichtung, welche bezüglich einer Schwenkachse S schwenkbar ist. Diese Schwenkachse S erstreckt sich hier senkrecht zur Figurenebene und auch parallel zu der Längsrichtung der (nicht gezeigten) Kunststoffvorformlinge bzw. Kunststoffbehältnisse. Bevorzugt ist die Schwenkachse von der (geometrischen Längsachse der Kunststoffvorformlinge wenigstens 2cm, bevorzugt wenigstens 3cm, bevorzugt wenigstens 4cm und besonders bevorzugt wenigstens 5cm entfernt. Bevorzugt ist die Schwenkachse von der (geometrischen Längsachse der Kunststoffvorformlinge höchstens 30cm, bevorzugt höchstens 25cm, bevorzugt höchstens 20cm und besonders bevorzugt höchstens 15cm entfernt.

Man erkennt, dass die Greifeinrichtung 62 und die Rückhalteeinrichtung bzw. das Rückhalteelement gemeinsam das Kunststoffbehältnis (nicht gezeigt) um einen Umfangswinkel umgeben, der größer ist als 180°, bevorzugt größer als 190°, bevorzugt größer als 200° und besonders bevorzugt größer als 210°. Bevorzugt ist dieser Winkel geringer als 320°, bevorzugt geringer als 310°, bevorzugt geringer als 300°, bevorzugt geringer als 290°, bevorzugt geringer als 280°, bevorzugt geringer als 270° und besonders bevorzugt geringer als 260°.

Die Position der Längsachse L der (nicht gezeigten) Kunststoffbehältnisse ist mit dem Buchstaben L gekennzeichnet. Man erkennt weiterhin, wie oben ausgeführt, dass der Umfangswinkel um den das Rückhalteelement das Kunststoffbehältnis umgibt größer ist als der Umfangswinkel um den die Greifeinrichtung das Kunststoffbehältnis umgibt.

Das Bezugszeichen 72 kennzeichnet, einen Ausleger, an welchem das Rückhalteelement 64 angeordnet ist. Das Bezugszeichen 74 kennzeichnet ein Befestigungselement, mit welchem die Greifeinrichtung 62 stationär, bevorzugt jedoch verstellbar an einem Träger befestigt ist. Durch diese Verstellbarkeit kann bevorzugt eine Anpassung an unterschiedliche Geometrien von Kunststoffvorformlingen oder allgemein Kunststoffbehältnissen erreicht werden.

Es wird darauf hingewiesen, dass sämtliche Merkmale, welche unter Bezugnahme auf das Verfahren beschrieben wurden, in entsprechender Weise auch für die Vorrichtung offenbart sind, was insbesondere bedeutet, dass die entsprechende Vorrichtung Einrichtungen aufweist, welche zur Durchführung der jeweiligen Verfahren geeignet und bestimmt sind. Weiterhin sind auch Merkmale, welche unter Bezugnahme auf die Vorrichtung beschrieben wurden entsprechend für das oder die Verfahren anwendbar. Dies bedeutet, dass die Verfahren unter Verwendung der entsprechenden Vorrichtungsmerkmale ausgeführt werden.

Es wird weiterhin darauf hingewiesen, dass sämtliche Merkmale, welche unter Bezugnahme auf die Vorrichtung zum Behandeln von Kunststoffbehältnissen beschrieben wurde auch in entsprechender weise für die Transportvorrichtung und die Halteeinrichtung. Gleiches gilt auch für Merkmale der Transporteinrichtung und der Halteeinrichtung, die entsprechend auch für die Vorrichtung zum Behandeln von Kunststoffbehältnissen offenbart sind.

Die Anmelderin behält sich vor sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

## Patentansprüche

1. Vorrichtung (1) zum Behandeln von Behältnissen, insbesondere von Kunststoffbehältnissen (10) und insbesondere von Kunststoffvorformlingen (10) mit einer Transporteinrichtung (2) zum Transportieren der Kunststoffbehältnisse (10) entlang eines vorgegebenen Transportpfads und mit wenigstens einer Beaufschlagungseinrichtung (4), welche dazu geeignet und bestimmt ist, die von der Transporteinrichtung (2) transportierten Kunststoffbehältnisse (10) mit einem fließfähigen Sterilisationsmedium und/oder einer Beschichtung und/oder einer elektromagnetischen Strahlung zu beaufschlagen, wobei die Transporteinrichtung (2) einen bewegbaren und bevorzugt um eine vorgegebene Drehachse drehbaren Transportträger (22) aufweist, an welchem eine Vielzahl von Halteeinrichtungen (6) zum Halten der Kunststoffbehältnisse (10) angeordnet ist,
**dadurch gekennzeichnet, dass**
die Halteeinrichtung (6) eine Greifeinrichtung (62) aufweist, welche dazu geeignet und bestimmt ist die Kunststoffbehältnisse wenigstens in einem ersten Abschnitt der Kunststoffbehältnisse zu kontaktieren sowie eine Rückhalteeinrichtung (64), welche dazu geeignet und bestimmt ist, die Kunststoffbehältnisse in einem zweiten Abschnitt der Kunststoffbehältnisse (10) zu kontaktieren, wobei der erste Abschnitt zum zweiten Abschnitt beabstandet ist, wobei die Greifeinrichtung relativ zur Transporteinrichtung feststehend ist und die Rückhalteeinrichtung relativ zur Transporteinrichtung beweglich ist.

2. Vorrichtung (1) nach Anspruch 1,
**dadurch gekennzeichnet, dass**
die Vorrichtung (1) einen Behandlungsraum, insbesondere einen Reinraum aufweist, innerhalb dessen die Behältnisse während ihrer Sterilisation transportiert werden.

3. Vorrichtung (1) nach wenigstens einem der vorangegangenen Ansprüche,
**dadurch gekennzeichnet, dass**
die Beaufschlagungseinrichtung derart angeordnet ist, dass sie im Wesentlichen eine Beaufschlagung eines Innenraums der Behältnisse mit dem fließfähigen Sterilisationsmedium ermöglicht wobei bevorzugt auch eine wenigstens teilweise Außensterilisation ermöglicht wird.

4. Transporteinrichtung (2) zum Transportieren von Behältnissen und insbesondere von Kunststoffvorformlingen wobei die Transporteinrichtung (2) einen um eine vorgegebene Drehachse bewegbaren und insbesondere drehbaren Transportträger (22) aufweist, an welchem eine Vielzahl von Halteeinrichtungen (6) zum Halten der Behältnisse (10) angeordnet ist,
**dadurch gekennzeichnet, dass**
die Halteeinrichtungen (6) eine Greifeinrichtung (62) ) aufweist, welche dazu geeignet und bestimmt ist die Kunststoffbehältnisse wenigstens in einem ersten Abschnitt der Kunststoffbehältnisse zu kontaktieren sowie eine Rückhalteeinrichtung (64), welche dazu geeignet und bestimmt ist, die Behältnisse in einem zweiten Abschnitt der Behältnisse (10) zu kontaktieren wobei der erste Abschnitt zum zweiten Abschnitt beabstandet ist, wobei die Greifeinrichtung relativ zur Transporteinrichtung und/oder dem zu transportierenden Behältnis feststehend ist und die Rückhalteeinrichtung relativ zur Transporteinrichtung und/oder dem zu transportierenden Behältnis beweglich ist um auf diese Weise die Behältnisse an der Greifeinrichtung zu halten, wobei die Rückhalteeinrichtung (64) ein zwischen einer Rückhalteposition und einer Freigabeposition bewegbares Rückhalteelement aufweist, wobei in der Rückhalteposition das Rückhalteelement das Behältnis und insbesondere einen Mündungsbereich des Behältnisses an der Greifeinrichtung hält und in der Freigabeposition das Behältnis aus einer an der Greifeinrichtung gebildeten Ausnehmung und insbesondere ohne Kontakt mit dem Rückhalteelement heraustreten kann.

5. Transporteinrichtung nach dem vorangegangenen Anspruch,
**dadurch gekennzeichnet, dass**
das Rückhalteelement zwischen der Rückhalteposition und der Freigabeposition um eine vorgegebene Schwenkachse schwenkbar ist wobei diese Schwenkachse bevorzugt in oder parallel zu der Längsrichtung der Behältnisse oder senkrecht zu der Längsrichtung der Behältnisse verläuft.

6. Transporteinrichtung nach wenigstens einem der vorangegangenen Ansprüche, **dadurch gekennzeichnet, dass**
die Transporteinrichtung wenigstens eine Antriebseinrichtung zum Bewegen und insbesondere Schwenken der Rückhalteelemente aufweist, wobei bevorzugt die Antriebseinrichtung eine Führungskurve aufweist, der gegenüber Führungsrollen abrollen, wobei bevorzugt jeder Halteeinrichtung wenigstens eine Führungsrolle zugeordnet ist.

7. Transporteinrichtung nach wenigstens einem der vorangegangenen Ansprüche, **dadurch gekennzeichnet, dass**
die Transporteinrichtung ein Schaltmittel aufweist, welches dazu geeignet ist, das Rückhaltemittel oder das Rückhalteelement zwischen der Rückhalteposition und der Freigabeposition zu schalten, wobei bevorzugt wenigstens ein magnetisches Fixiermittel vorgesehen ist welches dazu geeignet und bestimmt ist, das Rückhalteelement in einer der beiden Positionen zu fixieren.

8. Transporteinrichtung nach dem vorangegangenen Anspruch,
**dadurch gekennzeichnet, dass**
das magnetische Fixiermittel wenigstens einen Permanentmagneten aufweist wobei bevorzugt der wenigstens eine Permanentmagnet an dem Rückhalteelement oder dem Rückhaltemittel und insbesondere an einem schwenkbaren Rückhalteelement angeordnet ist und bevorzugt mit diesem Rückhalteelement schwenkbar ist.

9. Transporteinrichtung nach wenigstens einem der vorangegangenen Ansprüche, **dadurch gekennzeichnet, dass**
die Halteeinrichtungen (6) separat an dem Träger (22) angeordnet ist und insbesondere unabhängig voneinander von dem Träger (22) entfernbar und/oder unabhängig voneinander an dem Träger (22) anordenbar ist.

10. Transporteinrichtung nach wenigstens einem der vorangegangenen Ansprüche, **dadurch gekennzeichnet, dass**
die Greifeinrichtung das zu haltende Kunststoffbehältnis und/oder den zu haltenden ersten Abschnitt in einem ersten vorgegebenen Umfangswinkel umgibt und die Rückhalteeinrichtung das zu haltende Kunststoffbehältnis und insbesondere den zweiten Abschnitt in einem zweiten vorgegebenen Umfangswinkel umgibt, wobei bevorzugt der zweite Umfangswinkel größer ist als der erste Umfangswinkel.

11. Halteeinrichtung zum Halten von Behältnissen und insbesondere von Kunststoffvorformlingen wobei die Halteeinrichtung (6) eine Greifeinrichtung (62) aufweist, welche dazu geeignet und bestimmt ist die Kunststoffbehältnisse wenigstens in einem ersten Abschnitt der Kunststoffbehältnisse zu kontaktieren sowie eine Rückhalteeinrichtung (64), welche dazu geeignet und bestimmt ist, die Behältnisse in einem zweiten Abschnitt der Behältnisse (10), zu kontaktieren um auf diese Weise die Behältnisse an der Greifeinrichtung (62a, 62b) zu halten, wobei der erste Abschnitt zum zweiten Abschnitt beabstandet ist, wobei bevorzugt die Greifeinrichtung relativ zu dem Behältnis feststehend ist und die Rückhalteeinrichtung relativ zu dem Behältnis beweglich ist, wobei die Rückhalteeinrichtung (64) ein zwischen einer Rückhalteposition und einer Freigabeposition bewegbares Rückhalteelement aufweist, wobei in der Rückhalteposition das Rückhalteelement das Behältnis und insbesondere einen Mündungsbereich des Behältnisses (10) an der Greifeinrichtung und dem Rückhalteelement hält und in der Freigabeposition das Behältnis aus einer an der Greifeinrichtung gebildeten Ausnehmung und insbesondere ohne Kontakt mit dem Rückhalteelement heraustreten kann.

12. Halteeinrichtung nach dem vorangegangenen Anspruch,
**dadurch gekennzeichnet, dass**
das Rückhalteelement ein Element aufweist, welches in der Rückhalteposition in Kontakt mit dem Behältnis ist und welches bevorzugt in der Freigabeposition nicht in Kontakt mit dem Behältnis ist .

13. Halteinrichtung nach wenigstens einem der vorangegangenen Ansprüche
**dadurch gekennzeichnet, dass**
der erste Abschnitt und der zweite Abschnitt in der Längsrichtung der Behältnisse und/oder in der Umfangsrichtung der Behältnisse voneinander beabstandet sind.

14. Verfahren zum Behandeln und insbesondere zum Sterilisieren von Behältnissen, insbesondere von Kunststoffbehältnissen und insbesondere von Kunststoffvorformlingen, wobei eine Transporteinrichtung (2), welche einen um eine vorgegebene Drehachse drehbaren Transportträger (22) aufweist, an welchem eine Vielzahl von Halteeinrichtungen (6) zum Halten der Behältnisse (10) angeordnet ist, die Behältnisse entlang eines vorgegebenen Transportpfad transportiert,
wobei die Halteeinrichtungen (6) jeweils eine Greifeinrichtung (62) aufweisen, welche die Kunststoffbehältnisse wenigstens in einem ersten Abschnitt der Kunststoffbehältnisse zu kontaktieren sowie eine Rückhalteeinrichtung (64) die Behältnisse an einem zweiten Abschnitt der Behältnisse (10), kontaktiert wobei der erste Abschnitt zum zweiten Abschnitt beabstandet ist und wobei die Greifeinrichtung relativ zur Transporteinrichtung und/oder zum Behältnis feststehend ist und die Rückhalteeinrichtung relativ zur Transporteinrichtung und/oder zum Behältnis beweglich ist wobei die Rückhalteeinrichtung (64) ein zwischen einer Rückhalteposition und einer Freigabeposition bewegbares Rückhalteelement aufweist, wobei in der Rückhalteposition das Rückhalteelement das Behältnis und insbesondere einen Mündungsbereich des Behältnisses an der Greifeinrichtung hält und in der Freigabeposition das Behältnis aus einer an der Greifeinrichtung gebildeten Ausnehmung und insbesondere ohne Kontakt mit dem Rückhalteelement heraustreten kann.

15. Verfahren nach dem vorangegangenen Anspruch,
**dadurch gekennzeichnet, dass**
die Kunststoffvorformlinge während ihres Transports mit einem fließfähigen Sterilisationsmedium oder mit einer sterilisierenden Strahlung beaufschlagt werden, wobei bevorzugt auch ein Innenraum der Behältnisse sterilisiert wird und besonders bevorzugt auch ein Außenbereich der Behältnisse sterilisiert wird.
